(19) Europäisches Patentamt
European Patent Office
Office européen des brevets

(11) EP 1 357 180 A1

# (12) EUROPEAN PATENT APPLICATION

(43) Date of publication:
**29.10.2003 Bulletin 2003/44**

(51) Int Cl.[7]: **C12N 9/10**, A23L 1/03

(21) Application number: **02007933.1**

(22) Date of filing: **09.04.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Société des Produits Nestlé S.A.**
**1800 Vevey (CH)**

(72) Inventors:
- **Duboc, Phillipe**
  **1006 Lausanne (CH)**
- **Pridmore, Raymond-David**
  **1005 Lausanne (CH)**

(74) Representative: **Becker Kurig Straus**
**Patentanwälte**
**Bavariastrasse 7**
**80336 München (DE)**

Remarks:
The sequence listing, which is published as annex to the application documents, was filed after the date of filing. The applicant has declared that it does not include matter which goes beyond the content of the application as filed.

## (54) Levansucrase of lactobacillus johnsonii

(57) The present invention pertains to a new levansucrase gene derived from a food grade micro-organism. The present invention also relates to a polypeptide encoded by said gene and to its use in the manufacture of food material.

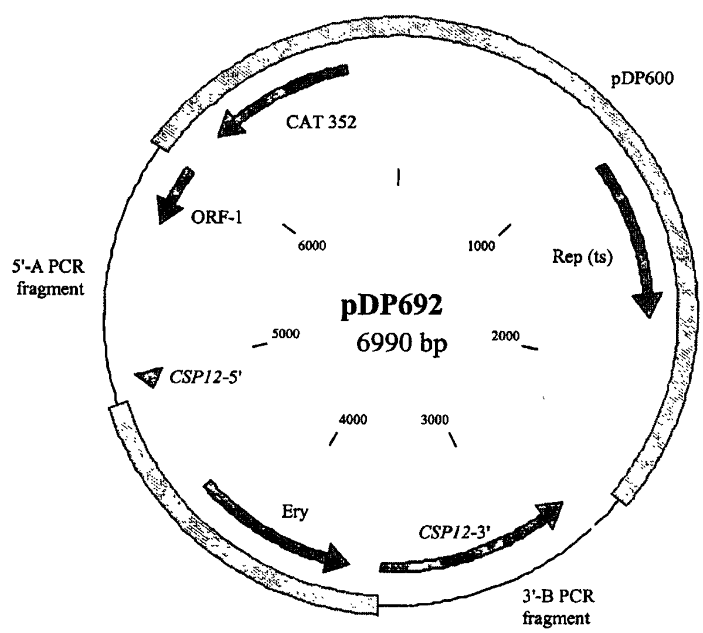


Figure 2. Schematic map of plasmid pDP692 used for the allelic replacement of the L. johnsonii La1 CSP12 gene.



Printed by Jouve, 75001 PARIS (FR)

## Description

The present invention pertains to a new levansucrase gene derived from a food grade micro-organism. The present invention also relates to a polypeptide encoded by said gene and to its use in the manufacture of food material.

In industry a number of different polysaccharides are utilized for a variety of purposes such as e.g. for maintaining moisture and viscosity of food, of cosmetics or of coating agents, for strengthening and texturing food materials, for improving the stability of food, and for use as low-calorie additives and as emulsion stabilizers.

To this end, high molecular weight polysaccharides produced by food grade bacteria are put to use, which generally exhibit a high dispensability in water.

Another class of polysaccharides utilized are synthesized by plants and consist largely or exclusively of fructose. These compounds include fructans, levans and inulin which are sweet tasting, water soluble polysaccharides that are not digested by humans and are hence considered as soluble dietary fibres.

Plant fructans, especially inulin are produced in many commonly consumed plants, including banana, onion, Jerusalem artichoke, leek, garlic, asparagus, wheat and chicory. Enriched inulin is also sold as a dietary supplement especially as a prebiotic sugar that promotes the growth of a few species of beneficial bacteria in the gut, i.e. Lactobacillus and Bifidobacteria, at the expense of less desirable species, such as Clostridia and Escherichia coli.

Inulin is naturally produced by approximately 15% of flowering plant species which are mainly concentrated in regions of seasonal drought or cold. This suggests that fructan production could be linked to an enhanced ability of these plants to survive under these stress conditions, an activity that is supported by the soluble nature of the polysaccharide allowing it to function as a osmo-protectant.

Fructans, especially levans are produced by some bacteria as well, where they seem to have an important role in dental biofilm formation. Fructan synthesis in bacteria is mediated by a fructosyltransferase or levansucrase enzymes that are secreted and usually attached to the bacterial cell surface. The levansucrase enzyme catalyses the following reaction:

$$\text{Sucrose + levan(n)} \rightarrow \text{glucose + levan(n+1)}$$

or, the more simple invertase activity:

$$\text{Sucrose + water} \rightarrow \text{glucose + fructose}$$

The resulting levans produced by bacteria usually contain many more fructose units as compared to those produced by plants, namely up to 100'000 compared to approximately 200 for plants.

In view of the increasing desire of people for a more healthy life, intensive investigations have been undertaken for the synthesis of oligosaccharides and useful glycosides having physiological activity.

Transferases of fructosyl groups, found in these efforts include the levan sucrase produced by Bacillus subtilis and beta -fructofuranosidase produced by the fungi such as Aspergillus niger, Penicillium oxalicum, Penicillium frequentans, Penicillium sp. K25 and the like. It has also been elucidated that xylsucrose and isomaltosyl fructosides synthesized via the activity of levan sucrase are characteristically cariostatic.

However, the levansucrases found so far are often difficult to handle and suffer from the shortcomings of a low productivity and poor heat resistance.

An object of the present invention therefore resides in providing a novel means to produce polysaccharides beneficial to humans upon ingestion.

This problem has been solved by providing a novel DNA sequence as identified by SEQ ID. NO. 1, or SEQ. ID. NO. 2, respectively, or functional parts thereof, all giving rise to a functional polypeptide exhibiting levansucrase activity. Likewise functional variants of SEQ. ID. NO. 1 or 2 or of functional parts thereof are comprised, that may be obtained by deleting, adding or substituting one or more nucleic acids and that have a degree of homology to SEQ ID. NO. 1, 2, or the respective functional parts, respectively, of more than 80 %.

In the context of the present invention a functional part of a nucleic acid of SEQ. ID. No. 1 or 2 shall be understood as a nucleic acid which may be obtained by deleting non-coding parts of SEQ. ID NO. 1 or 2 and/or sections of the open reading frame, that encode parts of the present polypeptide not participating in the enzymatic activity, e. g. the leader sequence and/or the anchoring domain.

In the figures,

Fig. 1 shows the genetic map of the L. johnsonii La1 CSP12 gene and flanking sequences;

Fig. 2 shows a schematic map of plasmid pDP651 used for the allelic replacement of the L. johnsonii La1 CSP12 gene;

Fig. 3 shows the levansucrase activity of L. johnsonii cells incubated in sucrose buffer at various temperatures;

Fig. 4 shows a graph indicating the molecular mass distribution of purified levan, produced with L. johnsonii, as determined on a Sephacryl S500 column; and

Figs. 5 shows the polypeptide sequence with the amino terminal secretion signal sequence and the gram positive cell wall anchor domain highlighted.

In the efforts to elucidate the genomic sequence of the probiotic strain Lactobacillus johnsonii La1, which is freely available from the Institute Pasteur under the accession no. CNCM I-1225, has revealed - inter alia - the presence of an open reading frame, which could not be annotated to other known functional gene sequences. According to the amino acid sequence derived from the nucleic acid sequence, the protein, which is termed CSP12 hereinafter (Cell Surface Protein 12), exhibits a leader sequence, and therefore seems to be secreted, and an anchoring domain, and therefore seems to be attached to the peptidoglycan on the bacterial cell wall.

In the studies leading to the present invention it has now been shown that the L. johnsonii La1 CSP-12 gene accounts for the synthesis of levan by this micro-organism, which gene seems to be a novel representative of levansucrases and exhibits an amino acid sequence, that essentially differs from known enzymes.

According to a preferred embodiment the degree of homology of a variant to the sequences shown in SEQ ID. No. 1, 2, or functional parts thereof, respectively, is more than 90%, more preferably more than 95 %, more preferred more than 97 %, or even more preferred more than 98 %.

According to another embodiment, the present invention provides for a novel levansucrase polypeptide as identified by SEQ ID. NO. 3 or functional parts thereof. A functional part is to be understood as a polypeptide, that lacks sections, not necessary for performing the enzymatic activity. Also comprised are functional variants thereof, which may be obtained by deleting, adding or substituting one or more amino acids, and which show a degree of homology to SEQ ID. NO. 3 or functional parts thereof, respectively, of more than about 80 %, more preferably more than 90 %, even more preferred more than 95 %, more preferred more than 97 %.

The nucleic acid as described herein may conveniently be utilized for expressing the novel levansucrase polypeptide in a host cell.

To this end, the nucleic acid may be included in an appropriate vector that allows expression of the desired polypeptide and the vector is introduced into a suitable host cell, such as Streptococcus thermophilus or Lactococcus lactis. Non-limiting examples for such vectors are e.g. pNZ124 (cf. Platteeuw et al., Use of the Escherichia coli β-glucuronidase (gusA) gene as a reporter gene for analyzing promoters in lactic acid bacteria, Appl. Environ. Microbiol. 60 (1994), 587-593) or pCK1 (cf. Gasson and Anderson, High copy number plasmid vectors for use in lactic streptococci FEMS Microbiol. Letters 30 (1985), 193-196). The vector will additionally contain sequences that enable its replication in the host cell selected and a selection marker to allows a stable transfer during propagation. Alternatively, the nucleic acid may be also be integrated into the host cell's chromosome by recombinant techniques, e.g. by means of homologous recombination.

Once integrated in the host cell, the said host will synthesize the polypeptide depending on the number of copies present in the cell and/or regulatory sequences driving the transcription of the gene.

According to an alternative embodiment the present invention also pertains to a micro-organism containing a nucleic acid sequence as described herein before, wherein the expression of the levansucrase gene has been modified.

A modification in the sense of the present invention designates any change of expression of the subjective polypeptide as compared to that found in the micro-organism utilized. Such modification may be achieved, by increasing or decreasing (if originally present) the copy number of the gene in the micro-organism, e.g. by means of a vector or by means of homologous recombination as described above. Alternatively, the expression level may also be modified by using regulatory sequences that promote the transcription of the gene operatively linked thereto. As an example, the strain L. johnsonii La1 may be modified such that the endogenous levansucrase gene is linked with a promotor different from the natural one and that increases/decreases the transcription rate. Such a promotor may be constitutive or inducible as desired. Examples for such promotors are e.g. L-lactate dehydrogenase or lactose operon promoters.

Alternatively a micro-organism according to the present invention may also be produced by conventional techniques of mutation and selection. In order to achieve this objective a micro-organism containing a nucleic acid as described herein, e.g. Lactobacillus johnsonii La1, is subjected to a mutagenic treatment and strains exhibiting a higher or lower production of levansucrase, as desired, are selected.

The polypeptide may be isolated by means well known in the art. To this end, the micro-organisms, potentially over-expressing the polypeptide, may be collected and the cell wall may be separated from the other cellular materials. In further steps, the levansucrase linked to the cell wall may be released therefrom and may be isolated by means of e.g. chromatographic measures.

The polypeptide has been found to have its strongest activity at a rather high temperature of about 42 °C producing up to 1.3 g/l levan, but still retains a significant activity at 4 °C. High activity is observed for pH values between 5 and 8, with pH 6 being the optimum. The present levansucrase produces a high molecular weight levan of approximately 2000 kDa that may be hydrolysed to fructose only. NMR-analysis showed that the levan produced with the present polypeptide is a linear polysaccharide with the structure $(\rightarrow 2)$-β-D-Fruf-$(6\rightarrow )_n$.

The levansucrase of the present invention may be utilized e.g. for producing levan in a medium containing glucose or sucrose, respectively. In this respect the polypeptide may be added to the medium as such, e.g. in form of a powder to be dissolved in the medium or in form of a liquid, or a micro-organism according to the present invention may be utilized for effecting the desired levan synthesis. Micro-organisms originally utilized in the preparation or processing of food materials are preferred, since they synthesize the objective compound during the process as such without involving additional steps.

The present invention also provides for a process of producing levan, which process involves the use of a levansucrase according to the present invention or a micro-organism according to the present invention.

The following examples illustrate the present invention without limiting it thereto.

## Examples

Materials and methods

L. johnsonii La1, that is freely available from the Institute Pasteur under the accession No. (CNCM-I-1225) was cultivated in Difco Lactobacillus MRS broth supplemented with 1 % glucose or 1 % sucrose at 37°C under anaerobic conditions.

Erythromycin and chloramphenicol were added at 4 μg/ml.

The Escherichia coli strain EC101 was grown aerobically at 37°C in Luria-Bertani (LB) broth or plated on Brain-Heart-Infusion (BHI) agar (Oxoid) supplemented with 150 μg/ml erythromycin and 30 μg/ml chloramphenicol.

The Lactococcus lactis strain MG1363 (cf. Gasson, Plasmid complements of *Streptococcus lactis* NCDO 712 and other lactic streptococci after protoplast-induced curing. J. Bacteriol. 154 (1983), 1-9) was grown under microaerophilic conditions in M17 broth supplemented with 0.5% glucose (GM17). Erythromycin was added at 4 μg/ml and 4 μg/ml chloramphenicol.

Plasmid pDP600-Ery was constructed by firstly exchanging the erythromycin resistance gene of pG+host9 (cf. Maguin et al., Efficient insertional mutagenesis in lactococci and other gram-positive bacteria. J. Bacteriol. 178 (1996), 931-5) with a modified chloramphenicol resistance gene (pDP600) and finally introducing the erythromycin gene into the middle of the cloning array. To achieve this objective, the chloramphenicol resistance gene was PCR amplified from plasmid pDP352 (cf. EP 93202513) using the oligonucleotide primers

D42 ($^{5'}$ATATATGAGCTCAGATCTGCTTTCATTTCACGCCTC$^{3'}$),

thus introducing SacI and BglII restriction sites (underlined), and

D43 ($^{5'}$ATATATGAGCTCAAAAAGCTAGTGATACTC$^{3'}$)

introducing a SacI restriction site (underlined).

PCR was performed using the Pwo thermostable polymerase on an Applied Biosystems 9700 thermocycler using the following amplification protocol: incubate at 95°C for 5 min, followed by 30 cycles of 95°C for 30 sec, 50°C for 30 sec, 68°C for 2 min and finally incubated at 68°C for 7 min before holding at 4°C. The amplicon was purified using the Millipore spin column system and the fragment eluted in 50 μl of TE buffer (10 mM Tris pH 8.0, 1 mM EDTA). A 25 μl sample was digested to completion with the restriction enzyme SacI, the desired fragment purified over a 1% agarose gel, extracted from the agarose using the Life Technologies Concert gel extraction system and eluted in a 50 μl volume of TE. This fragment was ligated into the E. coli vector pBluescript KS+ previously digested with the restriction enzyme SacI and dephosphorylated. The ligation was transformed into the E. coli strain EC101 and transformants were selected on LB plates supplemented with chloramphenicol. Individual colonies were screened by small scale plasmid preparation and digestion with the restriction enzymes Bg1II plus KpnI to identify a positive clone producing two fragments corresponding to the pBluescript KS+ vector and the chloramphenicol resistance gene. This plasmid was then digested with the restriction enzymes BglII plus KpnI, the chloramphenicol resistance gene gel purified and ligated into the vector pG+host9 previously digested with the restriction enzymes BamHI and KpnI plus dephosphorylated. The ligation was transformed into the E. coli strain EC101 and transformants were selected on LB plates supplemented with chloramphenicol. Individual colonies were screened by small scale plasmid preparation and digestion with the restriction enzymes to identify plasmid pDP600. A DNA fragment containing the erythromycin resistance gene flanked by convenient restriction sites was amplified from plasmid pVA838 (cf. Macrina et al., A cloning vector able to replicate in Escherichia coli and Streptococcus sanguis, Gene 19 (1982), 345-353) using the oligonucleotide primers

D135 (5'ATATAT<u>CTGCAG</u>AAAGTCCTACAATGCCG3')

introducing a PstI restriction site (underlined), and

D136 (5'ATATAT<u>GAATTC</u>TACAAATTCCCCGTAGGC3')

introducing an EcoRI restriction site (underlined). PCR amplification and purification was as described above and a sample digested with the restriction enzymes PstI plus EcoRI. After gel purification and extraction, the erythromycin resistance gene was ligated into plasmid pDP600 previously digested with the restriction enzymes PstI plus EcoRI plus dephosphorylated. The ligation was transformed into the E. coli strain EC101 and transformants selected on BHI plates supplemented with erythromycin. A correct plasmid was identified and named pDP600-Ery.

Transformation of L. johnsonii La1 was carried out as described here. Electro-competent L. johnsonii La1 were prepared as follows: An fresh overnight culture was used to inoculate at two percent a fresh tube of MRS broth containing 0.5 M sucrose and incubated overnight at 37 °C. This was used to inoculated at two percent 200 ml of fresh MRS broth containing 0.5 M sucrose and incubated until the culture reached an $OD_{600}$ of 0.6. Cells were harvested by centrifugation at 5'000 rpm at 4 °C for 10 min and the pellet suspended in 100 ml of 1 M sucrose plus 2,5 mM $CaCl_2$. The bacteria were again pelleted by centrifugation and the pellet suspended in 25 ml of the same solution. This wash step was repeated and the pellet finally suspended in 3.5 ml of the same solution containing a final concentration of 10 % glycerol. The competent cells were aliquoted and stored at -80 °C until use. Forty microliter aliquots of competent cells were mixed with 500 ng of plasmid and electro-transformed at 2.0 kV, 25 μF and 200 Ω. Cells were removed in 1 ml MRS medium containing 20 mM $MgCl_2$, 2 mM $CaCl_2$ plus 0.5 M sucrose and incubated for four hours at 32°C. Cells were plated on MRS-agar containing 4 μg/ml erythromycin at 32°C.

<u>Example 1</u>

**CSP12 gene identification**

During the sequencing of the Lactobacillus johnsonii La1 genome using the whole genome shotgun sequencing approach as described in Fleischmann et al., Whole-genome random sequencing and assembly of Haemophilus influenzae Rd., Science **269**(5223) (1995), 496-512, a variety of different open reading frames was found, most of which could be annotated to a biological activity due to a homology to polypeptides with known from other microorganisms.

Inter alia one open reading frame, that did not show a substantial homology to other known polypeptides could be located. Due to the presence of a Gram-positive cocci surface proteins 'anchoring' hexapeptide (LPXTG) (cf. Navarre and Schneewind, Surface proteins of Gram-positive bacteria and mechanisms of their targeting to the cell wall envelope. Microbiol. And Mol. Biol. Reviews; 63 (1999), 174-229) at the carboxyl, flanked by charged residues and followed by a hydrophobic, potentially membrane spanning domain with the protein finally terminating with charged residues, this open reading frame was considered to encode a putative cell wall anchored polypeptide. Therefore, the polypeptide was designated CSP-12 (Cell Surface Protein 12).

The CSP-12 gene and flanking sequences are shown in SEQ ID. NO. 1. The nucleic acid constituting the open reading frame is shown in SEQ. ID. NO. 2.

On the genomic level, the CSP12 gene has been found to be directly preceded by a predicted ribosome binding site (AGGAGGAGAAAAG). Further, the CSP12 gene is also preceded by a potential stem-loop structure with a predicted association energy of -18.6 kcal at approximately 200 base pairs upstream and directly followed after 3 base pairs by a potential stem-loop structure with a predicted association energy of -11.7 kcal(cf. Brendel et al., Terminators of transcription with RNA polymerase from *Escherichia coli*: what they look like and how to find them, J. Biomol. Struct. Dyn. 3 (1986), 705-723).

The polypeptide sequence derived from the open reading frame is shown in SEQ ID. NO.3 and contains 797 amino acids. At the amino terminus a potential secretion signal sequence is found which is predicted to cleaved between amino acids 36 and 37 (cf. Henrik Nielsen et al., Identification of prokaryotic and eukaryotic signal peptides and prediction of their cleavage sites, Protein Engineering **10** (1997), 1-6).

Example 2

**Knock out of the endogenous CSP-12 gene**

In order to elucidate the biological activity of the CSP-12 gene or the gene product, respectively, the CSP-12 gene in L. johnsonii was deleted and replaced by an erythromycin resistance gene as described below using plasmid pDP692. (cf. Biswas et al., High-efficiency gene inactivation and replacement system for Gram-positive bacteria, J. Bact. 175 (1993), 3628-3635).

To achieve this objective, a DNA fragment 5' to the CSP12 gene was PCR amplified from La1 genomic DNA using the oligonucleotide primers

D386 ($^{5'}$ATATATGAGCTCTTGGTGCCTACATGACTG$^{3'}$),

thus introducing a SacI restriction site (underlined), and

D387 ($^{5'}$ATATATGGATCCTGCTGTTGACAAGGTTCC$^{3'}$),

introducing a BamHI restriction site (underlined).

PCR amplification and purification was as described above and a sample digested with the restriction enzymes SacI plus BamHI and ligated into the plasmid pDP600-Ery previously digested with the restriction enzymes SacI plus BamHI and dephosphorylated. The ligation was transformed into the E. coli strain EC101 and transformants were selected on BHI plates supplemented with erythromycin. Individual colonies were screened by small scale plasmid preparation and restriction enzyme analysis and a positive plasmid was identified.

A DNA fragment 3' to the CSP12 gene was PCR amplified from La1 genomic DNA using the oligonucleotide primers

D667 ($^{5'}$ATATAT**GAATTC**CAAAGGTCTACAGCCCAC$^{3'}$)

introducing an EcoRI restriction site (underlined), and

D668 ($^{5'}$ATATAT**CTCGAG**TATAGATAGGTAGCACTC$^{3'}$)

introducing a XhoI restriction site (underlined). PCR amplification plus purification was as described above and a sample digested with the restriction enzymes EcoRI plus XhoI. After gel purification and extraction, the 3' CSP12 flanking fragment was ligated into the same plasmid with the 5' flanking fragment previously digested with EcoRI plus XhoI and dephosphorylated. The ligation was transformed into the E. coli strain EC101 and transformants selected on BHI plates supplemented with erythromycin, individual colonies screened by small scale plasmid preparation and restriction enzyme analysis and a positive plasmid identified. A correct plasmid was identified and named pDP692. Plasmid pDP692 was transformed and again extracted from the Lc. lactis strain MG1363 before transformation into L. johnsonii La1 and selection at 32°C.

The loop-in / loop-out gene replacement inactivation of the CSP12 gene was performed as described previously. L. johnsonii La1 containing plasmid pDP692 was incubated at 42°C with erythromycin selection. At this temperature, the replication protein of pDP600 is rendered inactive and these conditions select for the integration of pDP692 into the L. johnsonii La1 chromosome via homologous recombination and the flanking homologies. This selection is repeated three times until the colonies on selective plates are of uniform size. A single colony is then transferred to liquid culture containing erythromycin and cultivated for five serial passages at 42°C under anaerobic conditions. The culture was then streaked out onto a plate containing erythromycin to produce single colonies, which were then streaked onto fresh plates containing either erythromycin or chloramphenicol. L. johnsonii La1 containing the construct were selected due to their erythromycin resistance and their sensitivity to chloramphenicol.

The deletion of the endogenous CSP-12 gene has been confirmed by sequencing.

<u>Example 3</u>

**Biological activity of the CSP-12 gene**

Upon cultivation of the recombinant, knock-out L. johnsonii in a buffer containing sucrose it was noted that, in contrast to the non-modified L. johnsonii, the slightly milky color normally appearing in the culture broth during cultivation of the micro-organism did not become visible.

This milky color was attributed to the formation of exopolysaccharides (EPS) by L. johnsonii, in particular to the synthesis thereof on or at the cell surface of this micro-organism. Since the knock-out CSP-12 micro-organism lacked the capability of forming such EPS the biological activity of the present polypeptide was ascribed in general to an enzymatic activity on the cell surface of forming polysaccharides.

Moreover, this findings also confirmed that the enzymatic activity is present at cell surface as suggested by the presence of a putative leader sequence and a putative anchoring domain.

<u>Example 4</u>

**EPS Synthesis by L. johnsonii La1**

In order to elucidate, which sort of EPS is produced by the CSP-12 gene product, L. johnsonii La1 was grown for 24 hrs in MRS containing 20 g/l glucose and supplemented with various sucrose concentrations. Cells were collected by centrifugation and suspended in a buffer solution containing 100 mM sucrose, 200 mM sodium acetate, 50 mg/l $CaCl_2 \cdot 2H_2O$ and 0.2 g/l sodium azide. The pH of the buffer was adjusted to 5.5 with 2N HCl and the solution was incubated at various temperatures for 3-5 days.

For the purification of the EPS produced during cultivation, the cells were first removed by centrifugation. EPS present in the supernatant were precipitated overnight in ice-cold ethanol, recovered by centrifugation (20 min, 5000 rpm) and dissolved in distilled water prior to dialysis (1000 Da MW cut-off against distilled water overnight). Purified EPS were then freeze dried and stored as powder at room temperature.

EPS hydrolysis was achieved using trifluoroacetic acid (TFA) and the following conditions: 2 N for 150 min at 95°C; 1 N for 30 min at 50°C; and 0.5 N for 30 min at 50°C. After hydrolysis TFA was evaporated under a stream of $N_2$ at room temperature and the monosaccharides dissolved in distilled water. Monosaccharides were analyzed by HPLC (Aminex column HPX-87$H^+$ ; temperature, 35°C; mobile phase, 5 mM $H_2SO_4$; flow rate, 0.6 ml/min.). Standard solutions of glucose and fructose were subjected to similar hydrolysis conditions.

The molecular mass of the purified EPS was determined by gel filtration on Sephacryl S500 column at room temperature. The eluent was $NH_4HCO_3$ at 3 g/l with a flow of 0.4 ml/min. Fractions of 0.5 ml were collected and Total Neutral Sugar concentrations were determined by the phenol-sulfuric acid method (1 ml sample mixed with 1 ml of 5% w/v phenol solution and 5 ml of concentrated sulfuric acid; absorbance read at 485 nm). Dextrans of known average molecular mass (1.8 x $10^6$, 750'000 and 80'000 Da) were used as standards.

Only fructose was detected after TFA hydrolysis of the purified EPS. No other monosaccharides were found after acid hydrolysis under harsh conditions (2N TFA, 95°C for 2.5 hrs). Since levan is the only known polysaccharide synthesized after incubation in presence of sucrose, the polypeptide was identified as another representative of a levansucrase. Under mild conditions (0.5 and 1 N TFA at 50°C), fructose is not degraded and therefore levan can be detected as fructose after hydrolysis of weak β-2,6 bond between fructose moieties.

In order to show the substrate specificity of the levansucrase bound to La1 cells, raffinose (6-O-β-D-Galactopyranosyl-D-glucopyranosyl-β-D-fructofuranoside) was used as enzyme substrate. Raffinose is specifically converted to levan and melibiose (6-O-β-D-Galactopyranosyl-D-glucose) by levansucrase. Melibiose was detected by HPLC after incubation of the cell pellet in raffinose buffer for 5 days at 30°C, and the purified EPS that has been produced contained only fructose.

Cells grown in MRS medium with 20 g/l glucose and 17 g/l sucrose were recovered by centrifugation and suspended in sucrose buffer and incubated at temperatures of 4, 23, 30, 37 and 42°C for a period of 4 days. The amount of EPS formed was determined after ethanol precipitation and dialysis. The activity of levan-sucrase increased with temperature. The highest activity was obtained for 42°C.

The activity of levan-sucrase was calculated as the concentration of levan synthesized (expressed in μmol/1) per unit of time (in min). At 30°C, 2 g/l of levan accumulated within 4 days which corresponds to an enzyme activity of 2 μmol/min.

SEQUENCE LISTING

<110> Société des Produits Nestlé S.A.

<120> Levansucrase of Lactobacillus johnsonii

<130> 80363EP

<140> EP02007933.1
<141> 2002-04-09

<160> 14

<170> PatentIn version 3.1

<210> 1
<211> 2900
<212> DNA
<213> Lactobacillus johnsonii

<400> 1
ctcatctcca tagtaaaaag cctgtaagtt aaatacttac aggctttttt attatgtatt 60

aactgctatg tttaagattc aatagcagat tctgttttga aataaaactg ataatcgttt 120

aacattcagc actaataaag gattttcgct aaatctatac aatttttttct aaattttatt 180

tatttttta taaaaagatg ataggctttt agatgaaatg ttaaacgctt aacatgtagg 240

aggagaaaag atgttggaaa ataaaaatca taaaaagata tctttaagcg gaaaatcttt 300

gttaatggga accttgtcaa cagcagcaat tgtattaagt gcatcaactg caaatgctgc 360

gactattaat gcagacaatg ttaatgaaaa tcaaactgta gaagtaactg ctagttcagt 420

aaacaatgaa aataataagc aagtaactga aaaagatagt gcagataaaa gtactagtga 480

tgtggctgaa gatgctaaca ccaagaaatc aaacgaaaat acagaaacta cagaaaagaa 540

tactcaaaca gttgttacta atgcgccagt aagtgatgtg aaaaatacaa acacagttac 600

cgctgaaaca cctgttgata aagtagtaaa taatagtgat caaaagacaa ctaatgctgc 660

aactactgat actaaaaaag atgatgtaaa acaagttgaa aagaaagact cagtagataa 720

aacaaatgct gaggaaaata aagatagttc agtaaagcca gctgaaaatg ctactaaggc 780

tgaattaaag ggccaagtta aagatatcgt tgaagaatct ggtgttgata ctagcaagtt 840

aactaatgat caaattaatg aattaaataa aattaatttc tccaaagaag caaaaagtgg 900

tactcagtta acttacaacg actttaaaaa aattgctaaa actttaattg aacaagatgc 960

tcgttatgct attccattct tcaatgcaag taaaattaaa aatatgcctg ctgctaaaac 1020

acttgatgct caaagtggaa aagtagaaga tttggaaatt tgggattcat ggcctgttca 1080

agatgcaaaa actggttacg tatctaactg gaatggctac caattagtga ttggtatgat 1140

gggagttcca aacgtcaatg ataaccacat ttatcttctt tacaacaagt atggtgataa 1200

tgactttaat cattggaaga atgccggtcc tattttcggt ctaggtactc cagttattca 1260

acaatggtct ggatcagcaa ctttaaataa agatggctca attcaacttt actacactaa 1320

ggttgatact agtgataata atactaacca ccaaaaactc gctagtgcaa ctgtttactt 1380

aaatcttgaa aaagatcaag ataagatttc tattgctcat gttgacaacg accatattgt 1440

ctttgaaggt gatggttacc actaccaaac ttatgaccaa tggaaagaaa ctaacaaggg 1500

tgctgacaat atcgcaatgc gtgatgcaca cgtgattgat gatgataatg gtaatcgtta 1560

ccttgtgttt gaagcaagta ctggaaccga aaattatcaa ggtgatgatc aaatttatca 1620

atggttaaat tacggcggta ctaacaagga taatttaggt gatttcttcc aaattttatc 1680

taactccgat attaaagata gagctaaatg gtcaaacgct gcaattggta tcattaaatt 1740

aaatgatgat gttaagaatc caagtgttgc aaaggtctac agcccactta ttagtgcacc 1800

aatggtaagt gatgaaattg aacgccctga tgttgttaaa ttaggtaata agtattactt 1860

atttgctgct actagattaa accgtggtag taacgatgat gcttggatgg caactaacaa 1920

agcagttggt gataacgtag ctatgattgg ttatgtttct gataacttaa ctcatggtta 1980

tgttccattg aatgaatctg gcgttgtttt aactgcatct gtaccggcta actggcgtac 2040

tgcaacttat tcatactatg cagttccagt agaaggaaga gatgatcaac ttttaattac 2100

ttcatacatc actaatcgtg gtgaggttgc tggaaagggt atgcatgcaa cttgggcacc 2160

aagtttcttg ttacaaatta atccagataa cactactact gttttagcta aaatgactaa 2220

ccaaggggat tggatttggg atgatagtag tgaaaatcca gatatgatgg gtgtacttga 2280

aaaagatgct ccaaatagtg ctgcccttcc tggagaatgg ggaaaaccag ttgattggga 2340

tttaattggt ggatacaact tgaagccaca ccaacctgta actcctattc caaatgtacc 2400

aactactcct gaaaccccaa ccacaccaga taagccagag gtaccaacta cccctgaagt 2460

tccaaccact ccagaaactc caactccaga agctccaaag aatccagtta agaaaactag 2520

tcagtctaaa cttccaaagg ctggagataa aaatagcttt gcagcagttg ttttaggtgc 2580

tgtaagttca atattaggtg ctgttggttt aacaggtgtt tcaaaacgta aacgtaataa 2640

ttaaattaaa aaagatgagt tccgttgaac ttatcttttt ttgctataaa tataatttat 2700

cctaggatga aaaactgtaa attttctcaa aaatagcttt tttctataat gagttagaaa 2760

agtaatagag tgctacctat ctataggtca gaagtatatt taagtatgct tatcaaatat 2820

tagacaatta cttttcataa tttagactag atgtttgaaa gctttaataa aagaggaaat 2880

atatgcaaga aattaaagaa 2900

<210> 2
<211> 2394
<212> DNA
<213> Lactobacillus johnsonii

<400> 2

```
atgttggaaa ataaaaatca taaaaagata tctttaagcg gaaaatcttt gttaatggga      60
accttgtcaa cagcagcaat tgtattaagt gcatcaactg caaatgctgc gactattaat     120
gcagacaatg ttaatgaaaa tcaaactgta gaagtaactg ctagttcagt aaacaatgaa     180
aataataagc aagtaactga aaaagatagt gcagataaaa gtactagtga tgtggctgaa     240
gatgctaaca ccaagaaatc aaacgaaaat acagaaacta cagaaaagaa tactcaaaca     300
gttgttacta atgcgccagt aagtgatgtg aaaaatacaa acacagttac cgctgaaaca     360
cctgttgata aagtagtaaa taatagtgat caaaagacaa ctaatgctgc aactactgat     420
actaaaaaag atgatgtaaa acaagttgaa aagaaagact cagtagataa aacaaatgct     480
gaggaaaata aagatagttc agtaaagcca gctgaaaatg ctactaaggc tgaattaaag     540
ggccaagtta aagatatcgt tgaagaatct ggtgttgata ctagcaagtt aactaatgat     600
caaattaatg aattaaataa aattaatttc tccaaagaag caaaaagtgg tactcagtta     660
acttacaacg actttaaaaa aattgctaaa actttaattg aacaagatgc tcgttatgct     720
attccattct tcaatgcaag taaaattaaa aatatgcctg ctgctaaaac acttgatgct     780
caaagtggaa aagtagaaga tttggaaatt tgggattcat ggcctgttca agatgcaaaa     840
actggttacg tatctaactg gaatggctac caattagtga ttggtatgat gggagttcca     900
aacgtcaatg ataaccacat ttatcttctt tacaacaagt atggtgataa tgactttaat     960
cattggaaga atgccggtcc tattttcggt ctaggtactc cagttattca acaatggtct    1020
ggatcagcaa ctttaaataa agatggctca attcaacttt actacactaa ggttgatact    1080
agtgataata atactaacca ccaaaaactc gctagtgcaa ctgtttactt aaatcttgaa    1140
aaagatcaag ataagatttc tattgctcat gttgacaacg accatattgt ctttgaaggt    1200
gatggttacc actaccaaac ttatgaccaa tggaaagaaa ctaacaaggg tgctgacaat    1260
atcgcaatgc gtgatgcaca cgtgattgat gatgataatg gtaatcgtta ccttgtgttt    1320
gaagcaagta ctggaaccga aaattatcaa ggtgatgatc aaatttatca atggttaaat    1380
tacggcggta ctaacaagga taatttaggt gatttcttcc aaattttatc taactccgat    1440
attaaagata gagctaaatg gtcaaacgct gcaattggta tcattaaatt aaatgatgat    1500
gttaagaatc caagtgttgc aaaggtctac agcccactta ttagtgcacc aatggtaagt    1560
gatgaaattg aacgccctga tgttgttaaa ttaggtaata agtattactt atttgctgct    1620
actagattaa accgtggtag taacgatgat gcttggatgg caactaacaa agcagttggt    1680
gataacgtag ctatgattgg ttatgtttct gataacttaa ctcatggtta tgttccattg    1740
aatgaatctg gcgttgtttt aactgcatct gtaccggcta actggcgtac tgcaacttat    1800
tcatactatg cagttccagt agaaggaaga gatgatcaac ttttaattac ttcatacatc    1860
actaatcgtg gtgaggttgc tggaaagggt atgcatgcaa cttgggcacc aagtttcttg    1920
```

ttacaaatta atccagataa cactactact gttttagcta aaatgactaa ccaaggggat 1980

tggatttggg atgatagtag tgaaaatcca gatatgatgg gtgtacttga aaaagatgct 2040

ccaaatagtg ctgcccttcc tggagaatgg ggaaaaccag ttgattggga tttaattggt 2100

ggatacaact tgaagccaca ccaacctgta actcctattc caaatgtacc aactactcct 2160

gaaaccccaa ccacaccaga taagccagag gtaccaacta cccctgaagt tccaaccact 2220

ccagaaactc caactccaga agctccaaag aatccagtta agaaaactag tcagtctaaa 2280

cttccaaagg ctggagataa aaatagcttt gcagcagttg ttttaggtgc tgtaagttca 2340

atattaggtg ctgttggttt aacaggtgtt tcaaaacgta aacgtaataa ttaa 2394

<210> 3
<211> 797
<212> PRT
<213> Lactobacillus johnsonii

<400> 3

Met Leu Glu Asn Lys Asn His Lys Lys Ile Ser Leu Ser Gly Lys Ser
1 5 10 15

Leu Leu Met Gly Thr Leu Ser Thr Ala Ala Ile Val Leu Ser Ala Ser
20 25 30

Thr Ala Asn Ala Ala Thr Ile Asn Ala Asp Asn Val Asn Glu Asn Gln
35 40 45

Thr Val Glu Val Thr Ala Ser Ser Val Asn Asn Glu Asn Asn Lys Gln
50 55 60

Val Thr Glu Lys Asp Ser Ala Asp Lys Ser Thr Ser Asp Val Ala Glu
65 70 75 80

Asp Ala Asn Thr Lys Lys Ser Asn Glu Asn Thr Glu Thr Thr Glu Lys
85 90 95

Asn Thr Gln Thr Val Val Thr Asn Ala Pro Val Ser Asp Val Lys Asn
100 105 110

Thr Asn Thr Val Thr Ala Glu Thr Pro Val Asp Lys Val Val Asn Asn
115 120 125

Ser Asp Gln Lys Thr Thr Asn Ala Ala Thr Thr Asp Thr Lys Lys Asp
130 135 140

Asp Val Lys Gln Val Glu Lys Lys Asp Ser Val Asp Lys Thr Asn Ala
145 150 155 160

```
Glu Glu Asn Lys Asp Ser Ser Val Lys Pro Ala Glu Asn Ala Thr Lys
                165                 170                 175

Ala Glu Leu Lys Gly Gln Val Lys Asp Ile Val Glu Glu Ser Gly Val
            180                 185                 190

Asp Thr Ser Lys Leu Thr Asn Asp Gln Ile Asn Glu Leu Asn Lys Ile
        195                 200                 205

Asn Phe Ser Lys Glu Ala Lys Ser Gly Thr Gln Leu Thr Tyr Asn Asp
    210                 215                 220

Phe Lys Lys Ile Ala Lys Thr Leu Ile Glu Gln Asp Ala Arg Tyr Ala
225                 230                 235                 240

Ile Pro Phe Phe Asn Ala Ser Lys Ile Lys Asn Met Pro Ala Ala Lys
                245                 250                 255

Thr Leu Asp Ala Gln Ser Gly Lys Val Glu Asp Leu Glu Ile Trp Asp
            260                 265                 270

Ser Trp Pro Val Gln Asp Ala Lys Thr Gly Tyr Val Ser Asn Trp Asn
        275                 280                 285

Gly Tyr Gln Leu Val Ile Gly Met Met Gly Val Pro Asn Val Asn Asp
    290                 295                 300

Asn His Ile Tyr Leu Leu Tyr Asn Lys Tyr Gly Asp Asn Asp Phe Asn
305                 310                 315                 320

His Trp Lys Asn Ala Gly Pro Ile Phe Gly Leu Gly Thr Pro Val Ile
                325                 330                 335

Gln Gln Trp Ser Gly Ser Ala Thr Leu Asn Lys Asp Gly Ser Ile Gln
            340                 345                 350

Leu Tyr Tyr Thr Lys Val Asp Thr Ser Asp Asn Asn Thr Asn His Gln
        355                 360                 365

Lys Leu Ala Ser Ala Thr Val Tyr Leu Asn Leu Glu Lys Asp Gln Asp
    370                 375                 380

Lys Ile Ser Ile Ala His Val Asp Asn Asp His Ile Val Phe Glu Gly
385                 390                 395                 400

Asp Gly Tyr His Tyr Gln Thr Tyr Asp Gln Trp Lys Glu Thr Asn Lys
```

```
Asn Gln Gly Asp Trp Ile Trp Asp Asp Ser Ser Glu Asn Pro Asp Met
            660                 665                 670

Met Gly Val Leu Glu Lys Asp Ala Pro Asn Ser Ala Ala Leu Pro Gly
        675                 680                 685

Glu Trp Gly Lys Pro Val Asp Trp Asp Leu Ile Gly Gly Tyr Asn Leu
    690                 695                 700

Lys Pro His Gln Pro Val Thr Pro Ile Pro Asn Val Pro Thr Thr Pro
705                 710                 715                 720

Glu Thr Pro Thr Thr Pro Asp Lys Pro Glu Val Pro Thr Thr Pro Glu
                725                 730                 735

Val Pro Thr Thr Pro Glu Thr Pro Thr Pro Glu Ala Pro Lys Asn Pro
            740                 745                 750

Val Lys Lys Thr Ser Gln Ser Lys Leu Pro Lys Ala Gly Asp Lys Asn
        755                 760                 765

Ser Phe Ala Ala Val Val Leu Gly Ala Val Ser Ser Ile Leu Gly Ala
    770                 775                 780

Val Gly Leu Thr Gly Val Ser Lys Arg Lys Arg Asn Asn
785                 790                 795


<210>  4
<211>  36
<212>  DNA
<213>  Artificial Sequence

<220>
<223>  primer D42

<400>  4
atatatgagc tcagatctgc tttcatttca cgcctc                            36


<210>  5
<211>  30
<212>  DNA
<213>  Artificial Sequence

<220>
<223>  primer D43

<400>  5
atatatgagc tcaaaaagct agtgatactc                                   30


<210>  6
<211>  29
<212>  DNA
```

```
                405                 410                 415

Gly Ala Asp Asn Ile Ala Met Arg Asp Ala His Val Ile Asp Asp Asp
            420                 425                 430

Asn Gly Asn Arg Tyr Leu Val Phe Glu Ala Ser Thr Gly Thr Glu Asn
        435                 440                 445

Tyr Gln Gly Asp Asp Gln Ile Tyr Gln Trp Leu Asn Tyr Gly Gly Thr
    450                 455                 460

Asn Lys Asp Asn Leu Gly Asp Phe Phe Gln Ile Leu Ser Asn Ser Asp
465                 470                 475                 480

Ile Lys Asp Arg Ala Lys Trp Ser Asn Ala Ala Ile Gly Ile Ile Lys
                485                 490                 495

Leu Asn Asp Asp Val Lys Asn Pro Ser Val Ala Lys Val Tyr Ser Pro
            500                 505                 510

Leu Ile Ser Ala Pro Met Val Ser Asp Glu Ile Glu Arg Pro Asp Val
        515                 520                 525

Val Lys Leu Gly Asn Lys Tyr Tyr Leu Phe Ala Ala Thr Arg Leu Asn
    530                 535                 540

Arg Gly Ser Asn Asp Asp Ala Trp Met Ala Thr Asn Lys Ala Val Gly
545                 550                 555                 560

Asp Asn Val Ala Met Ile Gly Tyr Val Ser Asp Asn Leu Thr His Gly
                565                 570                 575

Tyr Val Pro Leu Asn Glu Ser Gly Val Val Leu Thr Ala Ser Val Pro
            580                 585                 590

Ala Asn Trp Arg Thr Ala Thr Tyr Ser Tyr Tyr Ala Val Pro Val Glu
        595                 600                 605

Gly Arg Asp Asp Gln Leu Leu Ile Thr Ser Tyr Ile Thr Asn Arg Gly
    610                 615                 620

Glu Val Ala Gly Lys Gly Met His Ala Thr Trp Ala Pro Ser Phe Leu
625                 630                 635                 640

Leu Gln Ile Asn Pro Asp Asn Thr Thr Thr Val Leu Ala Lys Met Thr
                645                 650                 655
```

<213> Artificial Sequence

<220>
<223> Primer D135

<400> 6
atatatctgc agaaagtcct acaatgccg 29

<210> 7
<211> 30
<212> DNA
<213> Artificial Sequence

<220>
<223> Primer D136

<400> 7
atatatgaat tctacaaatt ccccgtaggc 30

<210> 8
<211> 5
<212> PRT
<213> Unknown

<220>
<223> Gram-positive cocci surface proteins anchoring hexapeptide

<220>
<221> misc_feature
<222> (3)..(3)
<223> X = unknown or other

<400> 8

Leu Pro Xaa Thr Gly
1 5

<210> 9
<211> 13
<212> DNA
<213> Lactobacillus johnsonii

<400> 9
aggaggagaa aag 13

<210> 10
<211> 30
<212> DNA
<213> Artificial Sequence

<220>
<223> primer D386

<400> 10
atatatgagc tcttggtgcc tacatgactg 30

<210> 11

<211> 30
<212> DNA
<213> Artificial Sequence

<220>
<223> Primer D387

<400> 11
atatatggat cctgctgttg acaaggttcc 30

<210> 12
<211> 30
<212> DNA
<213> Artificial Sequence

<220>
<223> Primer D667

<400> 12
atatatgaat tccaaaggtc tacagcccac 30

<210> 13
<211> 30
<212> DNA
<213> Artificial Sequence

<220>
<223> Primer D668

<400> 13
atatatctcg agtatagata ggtagcactc 30

<210> 14
<211> 5
<212> PRT
<213> Unknown

<220>
<223> LPXAG signature

<220>
<221> misc_feature
<222> (3)..(3)
<223> X = unknown or other

<400> 14

Leu Pro Xaa Ala Gly
1 5

SEQID 1: CSP12 gene +/- 250 bp

CTCATCTCCATAGTAAAAAGCCTGTAAGTTAAATACTTACAGGCTTTTTTATTATGTATT
AACTGCTATGTTTAAGATTCAATAGCAGATTCTGTTTTGAAATAAAACTGATAATCGTTT
AACATTCAGCACTAATAAAGGATTTTCGCTAAATCTATACAATTTTTTCTAAATTTTATT
TATTTTTTTATAAAAAGATGATAGGCTTTTAGATGAAATGTTAAACGCTTAACATGTAGG
AGGAGAAAAGATGTTGGAAAATAAAAATCATAAAAAGATATCTTTAAGCGGAAAATCTTT
GTTAATGGGAACCTTGTCAACAGCAGCAATTGTATTAAGTGCATCAACTGCAAATGCTGC
GACTATTAATGCAGACAATGTTAATGAAAATCAAACTGTAGAAGTAACTGCTAGTTCAGT
AAACAATGAAAATAATAAGCAAGTAACTGAAAAAGATAGTGCAGATAAAAGTACTAGTGA
TGTGGCTGAAGATGCTAACACCAAGAAATCAAACGAAAATACAGAAACTACAGAAAAGAA
TACTCAAACAGTTGTTACTAATGCGCCAGTAAGTGATGTGAAAAATACAAACACAGTTAC
CGCTGAAACACCTGTTGATAAAGTAGTAAATAATAGTGATCAAAAGACAACTAATGCTGC
AACTACTGATACTAAAAAAGATGATGTAAAACAAGTTGAAAAGAAAGACTCAGTAGATAA
AACAAATGCTGAGGAAAATAAAGATAGTTCAGTAAAGCCAGCTGAAAATGCTACTAAGGC
TGAATTAAAGGGCCAAGTTAAAGATATCGTTGAAGAATCTGGTGTTGATACTAGCAAGTT
AACTAATGATCAAATTAATGAATTAAATAAAATTAATTTCTCCAAAGAAGCAAAAAGTGG
TACTCAGTTAACTTACAACGACTTTAAAAAAATTGCTAAAACTTTAATTGAACAAGATGC
TCGTTATGCTATTCCATTCTTCAATGCAAGTAAAATTAAAAATATGCCTGCTGCTAAAAC
ACTTGATGCTCAAAGTGGAAAAGTAGAAGATTTGGAAATTTGGGATTCATGGCCTGTTCA
AGATGCAAAAACTGGTTACGTATCTAACTGGAATGGCTACCAATTAGTGATTGGTATGAT
GGGAGTTCCAAACGTCAATGATAACCACATTTATCTTCTTTACAACAAGTATGGTGATAA
TGACTTTAATCATTGGAAGAATGCCGGTCCTATTTTCGGTCTAGGTACTCCAGTTATTCA
ACAATGGTCTGGATCAGCAACTTTAAATAAAGATGGCTCAATTCAACTTTACTACACTAA
GGTTGATACTAGTGATAATAATACTAACCACCAAAAACTCGCTAGTGCAACTGTTTACTT
AAATCTTGAAAAAGATCAAGATAAGATTTCTATTGCTCATGTTGACAACGACCATATTGT
CTTTGAAGGTGATGGTTACCACTACCAAACTTATGACCAATGGAAAGAAACTAACAAGGG
TGCTGACAATATCGCAATGCGTGATGCACACGTGATTGATGATGATAATGGTAATCGTTA
CCTTGTGTTTGAAGCAAGTACTGGAACCGAAAATTATCAAGGTGATGATCAAATTTATCA
ATGGTTAAATTACGGCGGTACTAACAAGGATAATTTAGGTGATTTCTTCCAAATTTTATC
TAACTCCGATATTAAAGATAGAGCTAAATGGTCAAACGCTGCAATTGGTATCATTAAATT
AAATGATGATGTTAAGAATCCAAGTGTTGCAAAGGTCTACAGCCCACTTATTAGTGCACC
AATGGTAAGTGATGAAATTGAACGCCCTGATGTTGTTAAATTAGGTAATAAGTATTACTT
ATTTGCTGCTACTAGATTAAACCGTGGTAGTAACGATGATGCTTGGATGGCAACTAACAA
AGCAGTTGGTGATAACGTAGCTATGATTGGTTATGTTTCTGATAACTTAACTCATGGTTA
TGTTCCATTGAATGAATCTGGCGTTGTTTTAACTGCATCTGTACCGGCTAACTGGCGTAC
TGCAACTTATTCATACTATGCAGTTCCAGTAGAAGGAAGAGATGATCAACTTTTAATTAC
TTCATACATCACTAATCGTGGTGAGGTTGCTGGAAAGGGTATGCATGCAACTTGGGCACC
AAGTTTCTTGTTACAAATTAATCCAGATAACACTACTACTGTTTTAGCTAAAATGACTAA
CCAAGGGGATTGGATTTGGGATGATAGTAGTGAAAATCCAGATATGATGGGTGTACTTGA
AAAAGATGCTCCAAATAGTGCTGCCCTTCCTGGAGAATGGGGAAAACCAGTTGATTGGGA
TTTAATTGGTGGATACAACTTGAAGCCACACCAACCTGTAACTCCTATTCCAAATGTACC
AACTACTCCTGAAACCCCAACCACACCAGATAAGCCAGAGGTACCAACTACCCCTGAAGT
TCCAACCACTCCAGAAACTCCAACTCCAGAAGCTCCAAAGAATCCAGTTAAGAAAACTAG
TCAGTCTAAACTTCCAAAGGCTGGAGATAAAAATAGCTTTGCAGCAGTTGTTTTAGGTGC
TGTAAGTTCAATATTAGGTGCTGTTGGTTTAACAGGTGTTTCAAAACGTAAACGTAATAA
TTAAATTAAAAAAGATGAGTTCCGTTGAACTTATCTTTTTTTGCTATAAATATAATTTAT
CCTAGGATGAAAAACTGTAAATTTTCTCAAAAATAGCTTTTTTCTATAATGAGTTAGAAA
AGTAATAGAGTGCTACCTATCTATAGGTCAGAAGTATATTTAAGTATGCTTATCAAATAT
TAGACAATTACTTTTCATAATTTAGACTAGATGTTTGAAAGCTTTAATAAAAGAGGAAAT
ATATGCAAGAAATTAAAGAA

SEQID 2: CSP12 gene

ATGTTGGAAAATAAAAATCATAAAAAGATATCTTTAAGCGGAAAATCTTTGTTAATGGGA
ACCTTGTCAACAGCAGCAATTGTATTAAGTGCATCAACTGCAAATGCTGCGACTATTAAT
GCAGACAATGTTAATGAAAATCAAACTGTAGAAGTAACTGCTAGTTCAGTAAACAATGAA
AATAATAAGCAAGTAACTGAAAAAGATAGTGCAGATAAAAGTACTAGTGATGTGGCTGAA
GATGCTAACACCAAGAAATCAAACGAAAATACAGAAACTACAGAAAAGAATACTCAAACA
GTTGTTACTAATGCGCCAGTAAGTGATGTGAAAAATACAAACACAGTTACCGCTGAAACA

Signal sequence

```
                                                                  60
MLENKNHKKISLSGKSLLMGTLSTAAIVLSASTANA ATINADNVNENQTVEVTASSVNNE

                                                                 120
NNKQVTEKDSADKSTSDVAEDANTKKSNENTETTEKNTQTVVTNAPVSDVKNTNTVTAET

                                                                 180
PVDKVVNNSDQKTTNAATTDTKKDDVKQVEKKDSVDKTNAEENKDSSVKPAENATKAELK

                                                                 240
GQVKDIVEESGVDTSKLTNDQINELNKINFSKEAKSGTQLTYNDFKKIAKTLIEQDARYA

                                                                 300
IPFFNASKIKNMPAAKTLDAQSGKVEDLEIWDSWPVQDAKTGYVSNWNGYQLVIGMMGVP

                                                                 360
NVNDNHIYLLYTSMVIMTLIIGRMPAPIFGLGTPVIQQWSGSATLNKDGSIQLYYTKVDT

                                                                 420
SDNNTNHQKLASATVYLNLEKDQDKISIAHVDNDHIVFEGDGYHYQTYDQWKETNKGADN

                                                                 480
IAMRDAHVIDDDNGNRYLVFEASTGTENYQGDDQIYQWLNYGGTNKDNLGDFFQILSNSD

                                                                 540
IKDRAKWSNAAIGIIKLNDDVKNPSVAKVYSPLISAPMVSDEIERPDVVKLGNKYYLFAA

                                                                 600
TRLNRGSNDDAWMATNKAVGDNVAMIGYVSDNLTHGYVPLNESGVVLTASVPANWRTATY

                                                                 660
SYYAVPVEGRDDQLLITSYITNRGEVAGKGMHATWAPSFLLQINPDNTTTVLAKMTNQGD

                                                                 720
WIWDDSSENPDMMGVLEKDAPNSAALPGEWGKPVDWDLIGGYNLKPHQPVTPIPNVPTTP

                                                                 780
ETPTTPDKPEVPTTPEVPTTPETPTPEAPKNPVKKT SQSKLPKAGDKNSFAAVVLGAVSS

                                                                 797
ILGAVGLTGVSKRKRNN
```

Gram-positive cocci surface proteins anchoring signature

Figure 5: Polypeptide sequence of the CSP12 protein. The predicted amino terminal secretion signal sequence is boxed and the gram positive cell wall anchor domain is circled at the carboxyl terminus while the highly conserved LPXTG (in this case LPXAG) signature is boxed.

CCTGTTGATAAAGTAGTAAATAATAGTGATCAAAAGACAACTAATGCTGCAACTACTGAT
ACTAAAAAAGATGATGTAAAACAAGTTGAAAAGAAAGACTCAGTAGATAAAACAAATGCT
GAGGAAAATAAAGATAGTTCAGTAAAGCCAGCTGAAAATGCTACTAAGGCTGAATTAAAG
GGCCAAGTTAAAGATATCGTTGAAGAATCTGGTGTTGATACTAGCAAGTTAACTAATGAT
CAAATTAATGAATTAAATAAAATTAATTTCTCCAAAGAAGCAAAAAGTGGTACTCAGTTA
ACTTACAACGACTTTAAAAAAATTGCTAAAACTTTAATTGAACAAGATGCTCGTTATGCT
ATTCCATTCTTCAATGCAAGTAAAATTAAAAATATGCCTGCTGCTAAAACACTTGATGCT
CAAAGTGGAAAAGTAGAAGATTTGGAAATTTGGGATTCATGGCCTGTTCAAGATGCAAAA
ACTGGTTACGTATCTAACTGGAATGGCTACCAATTAGTGATTGGTATGATGGGAGTTCCA
AACGTCAATGATAACCACATTTATCTTCTTTACAACAAGTATGGTGATAATGACTTTAAT
CATTGGAAGAATGCCGGTCCTATTTTCGGTCTAGGTACTCCAGTTATTCAACAATGGTCT
GGATCAGCAACTTTAAATAAAGATGGCTCAATTCAACTTTACTACACTAAGGTTGATACT
AGTGATAATAATACTAACCACCAAAAACTCGCTAGTGCAACTGTTTACTTAAATCTTGAA
AAAGATCAAGATAAGATTTCTATTGCTCATGTTGACAACGACCATATTGTCTTTGAAGGT
GATGGTTACCACTACCAAACTTATGACCAATGGAAAGAAACTAACAAGGGTGCTGACAAT
ATCGCAATGCGTGATGCACACGTGATTGATGATGATAATGGTAATCGTTACCTTGTGTTT
GAAGCAAGTACTGGAACCGAAAATTATCAAGGTGATGATCAAATTTATCAATGGTTAAAT
TACGGCGGTACTAACAAGGATAATTTAGGTGATTTCTTCCAAATTTTATCTAACTCCGAT
ATTAAAGATAGAGCTAAATGGTCAAACGCTGCAATTGGTATCATTAAATTAAATGATGAT
GTTAAGAATCCAAGTGTTGCAAAGGTCTACAGCCCACTTATTAGTGCACCAATGGTAAGT
GATGAAATTGAACGCCCTGATGTTGTTAAATTAGGTAATAAGTATTACTTATTTGCTGCT
ACTAGATTAAACCGTGGTAGTAACGATGATGCTTGGATGGCAACTAACAAAGCAGTTGGT
GATAACGTAGCTATGATTGGTTATGTTTCTGATAACTTAACTCATGGTTATGTTCCATTG
AATGAATCTGGCGTTGTTTTAACTGCATCTGTACCGGCTAACTGGCGTACTGCAACTTAT
TCATACTATGCAGTTCCAGTAGAAGGAAGAGATGATCAACTTTTAATTACTTCATACATC
ACTAATCGTGGTGAGGTTGCTGGAAAGGGTATGCATGCAACTTGGGCACCAAGTTTCTTG
TTACAAATTAATCCAGATAACACTACTACTGTTTTAGCTAAAATGACTAACCAAGGGGAT
TGGATTTGGGATGATAGTAGTGAAAATCCAGATATGATGGGTGTACTTGAAAAAGATGCT
CCAAATAGTGCTGCCCTTCCTGGAGAATGGGGAAAACCAGTTGATTGGGATTTAATTGGT
GGATACAACTTGAAGCCACACCAACCTGTAACTCCTATTCCAAATGTACCAACTACTCCT
GAAACCCCAACCACACCAGATAAGCCAGAGGTACCAACTACCCCTGAAGTTCCAACCACT
CCAGAAACTCCAACTCCAGAAGCTCCAAAGAATCCAGTTAAGAAAACTAGTCAGTCTAAA
CTTCCAAAGGCTGGAGATAAAAATAGCTTTGCAGCAGTTGTTTTAGGTGCTGTAAGTTCA
ATATTAGGTGCTGTTGGTTTAACAGGTGTTTCAAAACGTAAACGTAATAATTAA

## SEQID 3: CSP12 protein sequence

MLENKNHKKISLSGKSLLMGTLSTAAIVLSASTANAATINADNVNENQTVEVTASSVNNE
NNKQVTEKDSADKSTSDVAEDANTKKSNENTETTEKNTQTVVTNAPVSDVKNTNTVTAET
PVDKVVNNSDQKTTNAATTDTKKDDVKQVEKKDSVDKTNAEENKDSSVKPAENATKAELK
GQVKDIVEESGVDTSKLTNDQINELNKINFSKEAKSGTQLTYNDFKKIAKTLIEQDARYA
IPFFNASKIKNMPAAKTLDAQSGKVEDLEIWDSWPVQDAKTGYVSNWNGYQLVIGMMGVP
NVNDNHIYLLYNKYGDNDFNHWKNAGPIFGLGTPVIQQWSGSATLNKDGSIQLYYTKVDT
SDNNTNHQKLASATVYLNLEKDQDKISIAHVDNDHIVFEGDGYHYQTYDQWKETNKGADN
IAMRDAHVIDDDNGNRYLVFEASTGTENYQGDDQIYQWLNYGGTNKDNLGDFFQILSNSD
IKDRAKWSNAAIGIIKLNDDVKNPSVAKVYSPLISAPMVSDEIERPDVVKLGNKYYLFAA
TRLNRGSNDDAWMATNKAVGDNVAMIGYVSDNLTHGYVPLNESGVVLTASVPANWRTATY
SYYAVPVEGRDDQLLITSYITNRGEVAGKGMHATWAPSFLLQINPDNTTTVLAKMTNQGD
WIWDDSSENPDMMGVLEKDAPNSAALPGEWGKPVDWDLIGGYNLKPHQPVTPIPNVPTTP
ETPTTPDKPEVPTTPEVPTTPETPTPEAPKNPVKKTSQSKLPKAGDKNSFAAVVLGAVSS
ILGAVGLTGVSKRKRNN

**Claims**

1. A nucleic acid sequence as identified by SEQ ID. NO. 1, or parts thereof giving rise to a functional polypeptide, or functional variants thereof having a degree of homology of more than 80 %.

2. A nucleic acid as identified by SEQ ID. NO. 2, or parts thereof giving rise to a functional polypeptide, or variants thereof having a degree of homology of more than 80%.

3. The nucleic acid sequence according to claim 1 or 2, which has a degree of homology of more than 95 %.

4. A vector containing a nucleic acid according to any of the claims 1 to 3.

5. A polypeptide as identified by SEQ. ID. NO. 3 or functional parts thereof, or variants thereof having a degree of homology of more than 80 %.

6. The polypeptide according to claim 5, which has a degree of homology of more than 90 %.

7. A micro-organism containing a nucleic acid sequence according to any of the claims 1 to 4, the expression of the levansucrase gene having been modified.

8. The micro-organism according to claim 7, containing several copies of a nucleic acid according to any of the claims 1 to 3 or the regulatory region of the levansucrase gene being modified.

9. Use of a nucleic acid sequence according to any of the claims 1 to 3 for the synthesis of a polypeptide exhibiting levansucrase activity.

10. Use of a nucleic acid sequence according to any of the claims 1 to 3 for the synthesis of a recombinant micro-organism.

11. Use of a polypeptide according to claims 5 or 6, or a micro-organism according to any of the claims 7 or 8 for the preparation of food material.

12. Use of a nucleic acid according to claim 1 to 4 or a polypeptide according to claims 5 or 6, or a micro-organism according to claims 7 or 8 for the preparation of levan.

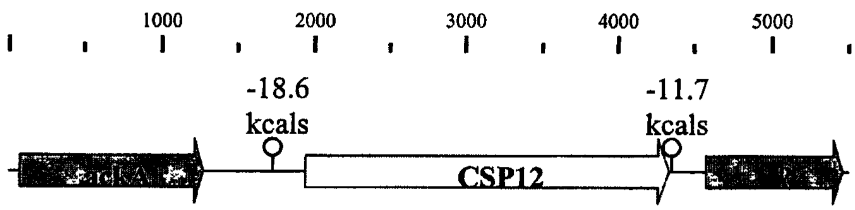


Figure 1 Gene map of *CSP12* and flanking genes.

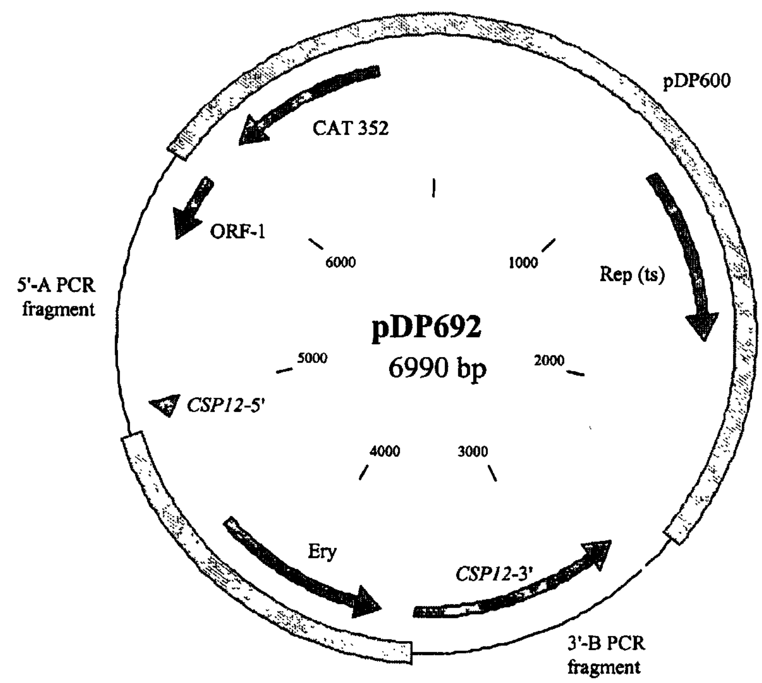


Figure 2. Schematic map of plasmid pDP692 used for the allelic replacement of the L. johnsonii La1 CSP12 gene.

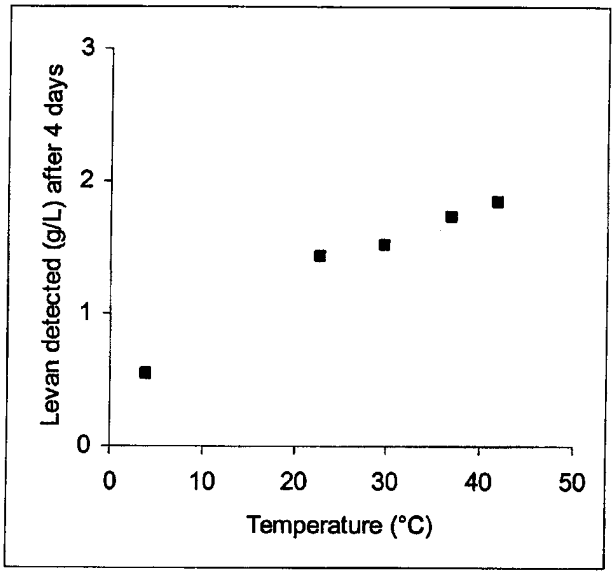


Figure 3: Levan synthesis by cells of La1 incubated in sucrose buffer at various temperatures. Cells were grown at 37°C in MRS containing 20 g/l glucose and 17 g/l sucrose. Levan was estimated from the amount of fructose determined by the sulfuric acid-phenol method.

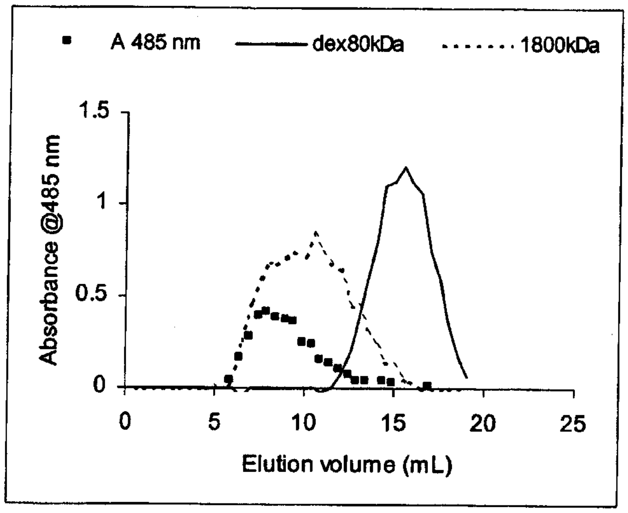


Figure 4: Molecular mass distribution of purified levan determined on Sephacryl S500 column.

European Patent Office

# EUROPEAN SEARCH REPORT

Application Number
EP 02 00 7933

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 01 90319 A (RAHAOUI HAKIM ;TNO (NL); DIJKHUIZEN LUBBERT (NL); GEEL SCHUTTEN GE) 29 November 2001 (2001-11-29)<br>* the whole document *<br>--- | 1-12 | C12N9/10<br>A23L1/03 |
| Y | WO 94 04692 A (INST GENBIOLOGISCHE FORSCHUNG ;ROEBER MANUELA (DE); GEIER GEBHARDT) 3 March 1994 (1994-03-03)<br>* page 4-5 *<br>--- | 1-12 | |
| E | WO 02 50311 A (NEESER JEAN-RICHARD ;VINCENT SEBASTIEN (CH); NESTLE SA (CH); CAVAD) 27 June 2002 (2002-06-27)<br>* abstract *<br>----- | 1-12 | |
| | | | TECHNICAL FIELDS SEARCHED (Int.Cl.7) |
| | | | C12N<br>C07K<br>A23L |

The present search report has been drawn up for all claims

| Place of search | Date of completion of the search | Examiner |
|---|---|---|
| MUNICH | 25 September 2002 | Young, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 00 7933

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2002

| Patent document cited in search report | Publication date | Patent family member(s) | Publication date |
|---|---|---|---|
| WO 0190319 A | 29-11-2001 | AU 6079101 A | 03-12-2001 |
| | | WO 0190319 A2 | 29-11-2001 |
| | | US 2002127681 A1 | 12-09-2002 |
| WO 9404692 A | 03-03-1994 | DE 4227061 A1 | 17-02-1994 |
| | | WO 9404692 A1 | 03-03-1994 |
| | | AU 682472 B2 | 09-10-1997 |
| | | AU 4946893 A | 15-03-1994 |
| | | EP 0663956 A1 | 26-07-1995 |
| | | JP 8500015 T | 09-01-1996 |
| | | US 5792923 A | 11-08-1998 |
| | | AU 5108498 A | 19-03-1998 |
| | | CA 2142308 A1 | 03-03-1994 |
| | | HU 70977 A2 | 28-11-1995 |
| | | US 6028249 A | 22-02-2000 |
| WO 0250311 A | 27-06-2002 | WO 0250311 A2 | 27-06-2002 |

EPO FORM P0459

For more details about this annex : see Official Journal of the European Patent Office, No. 12/82